# EUROPEAN PATENT SPECIFICATION

(11) **EP 2 861 696 B1**
(45) Date of publication and mention of the grant of the patent: **04.05.2022**
(21) Application number: 13731238.5
(22) Date of filing: 14.06.2013
(51) Int. Cl.: C10G 3/00, C10G 49/00, C10G 51/02, C10G 11/16, C10G 11/20

(54) **DIRECT CATALYTIC CRACKING OF CRUDE OIL BY A TEMPERATURE GRADIENT PROCESS**
VERFAHREN ZUR KATALYTISCHEN SPALTUNG VON ERDÖL NACH EINEM TEMPERATURGRADIENTEN-ADDITIONSVERFAHREN
PROCÉDÉ POUR LE CRAQUAGE CATALYTIQUE DE PÉTROLE BRUT PAR UN PROCÉDÉ EN PRÉSENCE D'UN GRADIENT DE TEMPÉRATURE

(30) Priority: 14.06.2012 US 201261659649 P
(43) Date of publication of application: 22.04.2015
(73) Proprietor: Saudi Arabian Oil Company, Dhahran 31311 (SA)
(72) Inventor: XU, Wei, Dhahran 31311 (SA); ABBA, Ibrahim, Dhahran 31311 (SA)
(74) Representative: Stafford, Jonathan Alan Lewis
(86) International application number: PCT/US2013/045781
(87) International publication number: WO 2013/188729

(56) References cited:
- WO-A1-98/05738
- US-A- 2 419 507
- US-A- 2 499 304
- US-A- 2 913 404
- US-A- 2 935 461
- US-A- 2 951 806
- US-A- 4 478 793

## Description

### BACKGROUND OF THE INVENTION

### 1. Field of the Invention

The field of invention relates to a method and a system for converting undistilled and unfractionated hydrocarbon materials into product hydrocarbons. More specifically, the field relates to systems and methods for converting hydrocarbon material, especially undistilled and unfractionated crude oil, into hydrocarbon product that comprises a mixture of light olefins and C6-8 aromatics.

### 2. Description of the Related Art

With the rise of global consciousness in relationship to our use of non-renewable resources, including crude oil, gas condensates and natural gas, as the basis for creating transportation fuels, the long-term upwards trend of gasoline and diesel fuel use may eventually level off and even decline in the next few decades, even in light of an increasing global population. The overall demand for petrochemicals, or chemicals sourced in part from hydrocarbon materials, will, however, continue to rise. Human society and its continuous technological advancement will require basic, intermediary and advanced petrochemical-based products, especially aromatics and polymers.

The modern petrochemical refinery design focuses on the production of motor fuels from virtually every fraction of the petroleum barrel. Natural gas, liquid condensates and crude oils have hydrocarbon fractions useful for making refinery intermediaries, including LPG, natural gasoline, naphtha, kerosene, atmospheric gas oil and vacuum gas oils. Downstream processes, in turn, convert these refinery intermediaries into fuel components for gasoline, jet, diesel and heavy transportation fuels. Refineries also make chemical feedstocks, including olefins like ethylene and propylene and aromatics like the xylenes as either primary or secondary process products. Current processes for making chemical feedstocks from fractions of crude oil and other sources of hydrocarbon materials, however, are energy, time and capital intensive.

Fractions of hydrocarbon materials act as feedstocks for chemical production. Common hydrocarbon feedstocks fractions include ethane, LPG, naphtha, and gas oils. Chemical manufacturers use these fractions to produce "light" olefins (for example, ethylene and propylene) for downstream use. Two primary techniques for creating olefins from alkanes includes steam cracking processes and fluidized catalytic cracking (FCC) processes. "Cracking" is the breaking of carbon-carbon bonds present in a hydrocarbon material.

Steam or "thermal" cracking is a non-catalytic petrochemical conversion process. Steam cracking can produce simple olefins, including ethylene, propylene, and butenes; butadiene; and aromatics, including the BTEX compounds (benzene, toluene, ethyl benzene and the three isomers of xylenes) from a variety of liquid and vaporous hydrocarbon feedstocks, including ethane and heavy naphtha. For example, mixing a naphtha feedstock with superheated steam, which acts both as a diluent and as the provider of thermal energy, and introducing it into a reforming furnace operating in a range of about 700-900 °C produces olefins and hydrogen form from the cracking of the paraffinic materials in the naphtha feedstock.

Thermal cracking is a widely recognized and used process for producing light olefins; however, it is also one of the greatest energy-consuming processes in the petrochemical industry. The pyrolysis section of a steam cracker can consume about 65% of process energy for a facility. It also can account for about 75% of the total energy loss in the process. Avoiding the use of purely thermal-based processes can significantly reduce the ongoing energy requirements for producing olefins and aromatics.

A fluid catalytic cracking (FCC) process utilizes both thermal and catalytic process reaction processes. The process employs a catalyst mixed and suspended in a rising flow of vaporized feed hydrocarbons such that a fluidized bed of solid catalyst forms. The pre-heated hydrocarbon feed, usually a gas oil, sprays into the base of a riser pipe via feed nozzles. Hot, fluidized catalyst contacts the hydrocarbon feed such that the feed flashes into a vapor. In the vapor state, the hydrocarbons catalytically crack into olefins and aromatics as well as lower carbon paraffins and cycloparaffins. The cracked hydrocarbon product egresses from the FCC unit. Downstream systems cool and partition the cracked product from hydrogen gas to fuel oils, which then are distributed to various other systems for further processing. A byproduct of the catalytic cracking process is "coke", which is a form of carbon residue, on the catalyst. A second unit in the FCC process gathers the coked or "spent" catalyst, uses steam and then an oxygen source, and "regenerates" the catalyst for use again in the FCC. The FCC process preheats the catalyst and reintroduces the regenerated catalyst into the riser, repeating the cracking cycle.

"Coke" and derivative terms (that is, "coking") refer to high-molecular weight carbonaceous solids, predominantly but not exclusively made of carbon atoms. Coke residue most often forms from the stripping of hydrogen from the introduced hydrocarbon materials at the high processing temperatures, including the condensation of polynuclear aromatics and asphaltine-like materials. The coke is removed after exposing it to oxygen and forming carbon oxides.

The FCC process is much more energy efficient than steam cracking; however, the FCC process can also only handle certain types of contaminants, including coke, acids and specific heavy metals. Nitrogen-bearing and sulfur-bearing compounds are known FCC catalyst poisons.

US 2951806 A discloses a process for the catalytic conversion of hydrocarbon charge stocks to produce high-octane gasoline and light distillate fuels, and is particularly directed to improvements in the so-called moving-bed type of process wherein hot granular catalyst having an average diameter of about 2-13 mm is continuously circulated as a compact moving mass through a treating system having alternate stages of hydrocarbon conversion and catalyst regeneration.

US 2935461 A discloses a process for the catalytic cracking of heavy charge stocks to produce high-octane gasoline and light distillate fuels, and is particularly directed to the so-called moving-bed type of process in which hydrocarbons contact hot granular catalyst of a particle-size in the range of about 2-13 mm while the latter gravitates through the reaction zone as a compact moving bed, and in which the catalyst is continuously circulated through alternate stages of reaction and regeneration.

US 2419507 A discloses apparatus for and methods of conducting catalytic conversion of hydrocarbons and of regenerating the catalysts used for the conversion, invention residing in each of the single operations and the apparatus therefor, as well as, in combinations of the single operations and apparatus therefor which afford continuous processes and means for carrying out same.

US 2913404 A discloses a process and apparatus for handling granular solids and in particular relates to the handling of granular solid materials in an improved solids-fluid contact system, in particular the high temperature conversion of hydrocarbons wherein a hydrocarbon feed stock is introduced, at least partly in the liquid phase, into contact with the stream of granular solid contact material. Preferably this contact material comprises an adsorptive hydrocarbon conversion catalyst in contact with which the hydrocarbon, at least partly in the liquid phase, is reacted and converted to produce hydrocarbon products of higher volatility and lower boiling range.

US 4478793 A discloses a radial flow reactor design which allows the maintenance of a temperature profile along the height of a catalyst bed which descends through the reactor by gravity flow. Two feed inlet conduits are provided on the inlet side of the annular catalyst bed, with the inlets being divided by a porous flow control and distribution means. The inlet streams have different temperatures and the distribution means produces a changing admixture rate between the two streams along the bed.

US 2499304 A discloses a method and apparatus for conversion of high boiling liquid hydrocarbons to lower boiling products in the presence of a moving, substantially compact mass of particle form solid contact material.

The design and function of both the thermal and the FCC processes cannot handle full boiling point range crude oil as a primary feed. There are two major challenges for cracking full boiling point range hydrocarbon feeds that prohibit the use of either of these processes. Rapid catalyst deactivation due to coke formation and catalyst contamination from heavy metals and poisons present in the unfractionated hydrocarbon feed pose difficulty to using catalyst-based systems. The wide range of temperatures in which the "crackable" crude oil components crack poses a problem of process control and long-term system integrity (that is, temperature/time based stress failure) for pure thermal processes.

### SUMMARY OF THE INVENTION

A direct catalytic cracking system that is operable to convert an undistilled and unfractionated hydrocarbon material into a hydrocarbon product comprising a mixture of light olefins and C6-8 aromatics using a direct cracking catalyst is provided, the direct catalytic cracking system comprising: a moving catalyst bed reactor that is operable to receive a moving bed reactor feed comprising the undistilled and unfractioned hydrocarbon material and to pass a hydrocarbon product comprising a mixture of light olefins and C6-8 aromatics, that has more than one catalytic reaction zones, where each catalytic reaction zone is operable to receive the direct cracking catalyst, to contain an amount of the direct cracking catalyst in the form of a moving catalyst bed and to pass a spent direct cracking catalyst such that it egresses from the moving catalyst bed reactor, that is operable to permit the intermingling of hydrocarbons with the direct cracking catalyst in the moving catalyst bed along at least a portion of the operative length of the moving catalyst bed reactor, and having means for maintaining an internal temperature profile for the moving catalyst bed reactor along its operative length; and a catalyst regeneration system that couples to the moving catalyst bed reactor such that the direct catalytic cracking system is operable to introduce the direct cracking catalyst from the catalyst regeneration system to each catalytic reaction zone and is operable to introduce the spent direct cracking catalyst from each catalytic reaction zone to the catalyst regeneration system, and that is operable to convert introduced the spent direct cracking catalyst into the direct cracking catalyst using a source of oxygen, wherein the internal temperature profile is maintained as more than one fixed temperature zone between a position of introduction for the moving bed reactor feed and a position of passing for the hydrocarbon product, where a first fixed temperature zone having a first temperature is positioned proximate to the position of introduction and a second fixed temperature zone having a second temperature is positioned proximate to the position of passing, wherein the apparatus is configured such that the second temperature is greater than the first temperature and the temperature increases from the first temperature to the second temperature along the operative length; and wherein each catalytic reaction zone is associated with one of the fixed temperature zones and adjacent catalytic reaction zones fluidly couple with one another through at least one intermediary solids separation device. Hydrocarbon product includes light olefins and C6-8 aromatics, which includes benzene, the xylenes, ethyl benzene and toluene. The direct catalytic cracking system includes a moving catalyst bed reactor having one or more catalytic reaction zones and is operable to maintain the internal temperature of the reactor. The direct catalytic cracking system also includes a catalyst regeneration system.

A method of converting undistilled and unfractionated hydrocarbon material into a hydrocarbon product using a direct catalytic cracking system is provided, the method comprising the steps of: introducing a moving bed reactor feed into a moving catalyst bed reactor of the direct catalytic cracking system such that the moving bed reactor feed intermingles with a direct cracking catalyst contained in a catalytic reaction zone within the moving catalyst bed reactor, where the moving bed reactor feed comprises steam and an undistilled and unfractionated hydrocarbon material; introducing the direct cracking catalyst into the catalytic reaction zone of the moving catalyst bed reactor; and operating the direct catalytic cracking system such that an internal temperature profile is maintained along an operative length of the moving catalyst bed reactor, such that a moving catalyst bed comprising the direct cracking catalyst is maintained in the catalytic reaction zone, such that the hydrocarbon material converts in the presence of the direct cracking catalyst into a product hydrocarbon and the direct cracking catalyst converts into a spent direct cracking catalyst in the catalytic reaction zone, such that the spent direct cracking catalyst passes from the catalytic reaction zone to a catalyst regeneration system, and such that the hydrocarbon product is produced from the moving catalyst bed reactor, the hydrocarbon product comprising light olefins and C6-8 aromatics; where the direct catalytic cracking system includes the moving catalyst bed reactor and the catalyst regeneration system, and where the moving catalyst bed reactor has the catalyst reaction zone and is operable to maintain the internal temperature profile for the moving catalyst bed reactor along its operative length, wherein the internal temperature profile is maintained as more than one fixed temperature zone between a position of introduction for the moving bed reactor feed and a position of passing for the hydrocarbon product, where a first fixed temperature zone having a first temperature is positioned proximate to the position of introduction and a second fixed temperature zone having a second temperature is positioned proximate to the position of passing, and where the second temperature is greater than the first temperature, wherein the temperature increases from the first temperature to the second temperature along the operative length.

Direct conversion of hydrocarbon materials into a hydrocarbon product useful as chemical feedstocks does not require pre-fractionation or purification of the hydrocarbon material. Direct conversion also avoids the capital expense of large fractionation facilities and the energy required to operate them to produce fuels. Direct conversion into useful chemicals such as olefins and aromatics ensures the best and most valuable use for the non-renewable hydrocarbon material.

The direct catalytic cracking system includes the moving catalyst bed reactor that contains the direct hydrocarbon cracking catalyst. The moving catalyst bed reactor is an improvement over a static bed catalyst system in that the system treats catalyst for poisons and coke while continuing operations. The moving catalyst bed reactor also provides for prolonged contact by the catalyst with the hydrocarbon material. The longer contact time permits operating the moving catalyst bed reactor at lower temperatures versus a FCC-type systems. Lower operating temperatures in and of itself assists in avoiding coke formation on the direct cracking catalyst.

The direct cracking catalyst improves cracking efficiency and reduces the formation of coke on the catalyst at the operating conditions of the moving catalyst bed reactor. Both the rate of coke formation and the cracking rate of a particular hydrocarbon specie are a function of temperature. The lower temperature boiling point fractions of hydrocarbons, for example, ethane and butane, have fewer tendencies to coke; however, they require higher temperatures to crack into olefins. High temperature boiling fractions, including those contained in atmospheric and vacuum oil gases, easily crack into smaller compounds and olefins; however, the coking tendencies are also higher.

Operation of the direct catalytic cracking system regularly purges and regenerates the cracking catalyst of coke and metal impurities.

### BRIEF DESCRIPTION OF THE DRAWINGS

These and other features, aspects, and advantages of the present invention are better understood with regard to the following Detailed Description of the Preferred Embodiments, appended Claims, and accompanying Figures, where:
Figure 1 is a general flow schematic of one embodiment of a direct catalytic cracking system of the current invention; and
Figure 2 is a general flow schematic of another direct catalytic cracking system.

In the appended Figures, similar components or features, or both, may have the same or similar reference labels.

### DETAILED DESCRIPTION OF THE PREFERRED EMBODIMENTS

The Specification, which includes the Summary of Invention, Brief Description of the Drawings and the Detailed Description of the Preferred Embodiments, and the appended Claims refer to particular features (including process or method steps) of the invention. Those of skill in the art understand that the invention includes all possible combinations and uses of particular features described in the Specification. Those of skill in the art understand that the invention is not limited to or by the description of embodiments given in the Specification.

In interpreting the Specification and appended Claims, all terms should be interpreted in the broadest possible manner consistent with the context of each term. All technical and scientific terms used in the Specification and appended Claims have the same meaning as commonly understood by one of ordinary skill in the art to which this invention belongs unless defined otherwise.

As used in the Specification and appended Claims, the singular forms "a", "an", and "the" include plural references unless the context clearly indicates otherwise. The verb "comprises" and its conjugated forms should be interpreted as referring to elements, components or steps in a non-exclusive manner. The referenced elements, components or steps may be present, utilized or combined with other elements, components or steps not expressly referenced. The verb "couple" and its conjugated forms means to complete any type of required junction, including electrical, mechanical or fluid, to form a singular object from two or more previously non-joined objects. If a first device couples to a second device, the connection can occur either directly or through a common connector. "Optionally" and its various forms means that the subsequently described event or circumstance may or may not occur. The description includes instances where the event or circumstance occurs and instances where it does not occur. "Operable" and its various forms means fit for its proper functioning and able to be used for its intended use. "Associated" and its various forms means something connected with something else because they occur together or that one produces the other.

Spatial terms describe the relative position of an object or a group of objects relative to another object or group of objects. The spatial relationships apply along vertical and horizontal axes. Orientation and relational words including "above" and "below", "up" and "down", "upstream" and "downstream" and other like terms are for descriptive convenience and are not limiting unless otherwise indicated.

Where the Specification or the appended Claims provide a range of values, it is understood that the interval encompasses each intervening value between the upper limit and the lower limit as well as the upper limit and the lower limit. The invention encompasses and bounds smaller ranges of the interval subject to any specific exclusion provided.

Where the Specification and appended Claims reference a method comprising two or more defined steps, the defined steps can be carried out in any order or simultaneously except where the context excludes that possibility.

### Figure 1

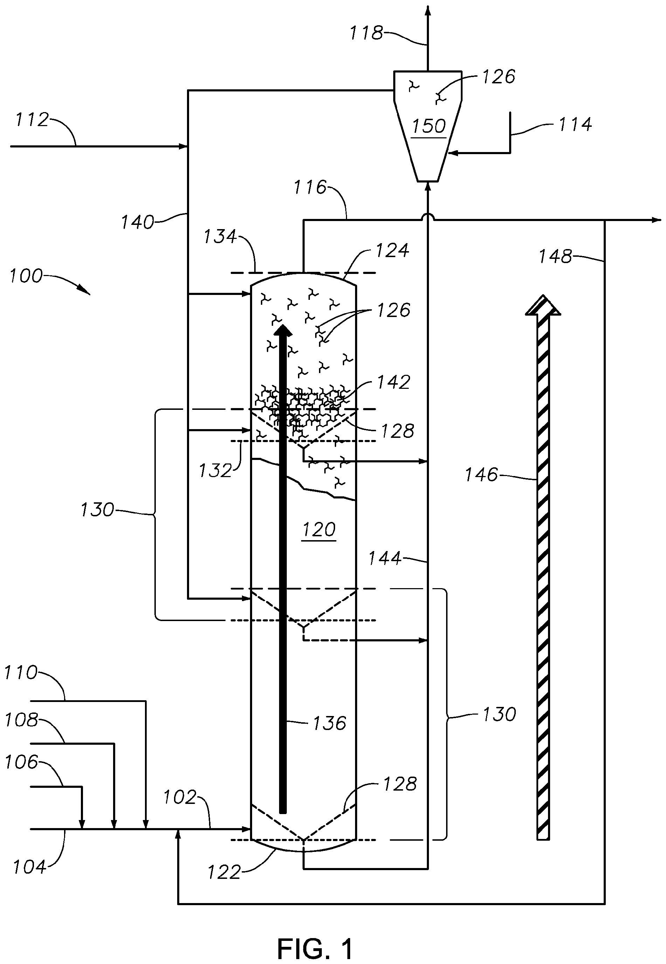

Figure 1 is a general flow schematic of an embodiment of a direct catalytic cracking system. Figure 1 and its description facilitate a better understanding of a direct catalytic cracking system and method of use. In no way should Figure 1 limit or define the scope of the invention.

Direct catalytic cracking system 100 is operable to receive the introduction of moving bed reactor feed 102, which includes the contents of hydrocarbon material feed 104, steam feed 106, optional hydrogen feed 108 and optional oxygen feed 110. Direct catalytic cracking system 100 is also operable to receive the introduction of fresh direct cracking catalyst feed 112. The introduction of heated air 114, which is a source of oxygen, supports the combustion of coke and cracking residues. Direct catalytic cracking system 100 also has two major product streams: product hydrocarbons 116 and catalyst residue off-gas 118.

Direct catalytic cracking system 100 includes moving catalyst bed reactor 120. Figure 1 shows moving catalyst bed reactor 120 in partial internal reveal. Moving catalyst bed reactor 120 has bottom 122 and top 124. Internally, moving catalyst bed reactor 120 contains an amount of direct cracking catalyst 126 (represented by triforms; not actual size) operational at conditions maintained in moving catalyst bed reactor 120 for converting hydrocarbon materials into smaller hydrocarbons, including paraffins, olefins and aromatics.

Moving catalyst bed reactor 120 has several internal perforated collection funnels 128. Internal perforated collection funnels 128 are operable to collect direct cracking catalyst 126 for recycle. Internal perforated collection funnels 128 are operable to permit fluids to pass through, including hydrocarbon material, but not solids, including heterogeneous catalyst. Due to gravity, direct cracking catalyst 126 aggregates on top of and in internal perforated collection funnels 128 until removed from moving catalyst bed reactor 120. In common practice, direct cracking catalyst 126 fills most of the working volume of moving catalyst bed reactor 120, especially in catalytic reaction zone 130.

Internal perforated collection funnels 128 generally demarcate the boundaries of the reaction zones. For example, in moving catalyst bed reactor 120 for catalytic reaction zone 130, a lower bound exists at reaction zone lower bound 132 (represented by dotted line), which represents the lowest vertical point for the reaction zone where the catalytic cracking reaction can occur. An upper bound exists at reaction zone upper bound 134 (represented by dashed line) at the highest point along internal perforated collection funnel 128 for the uppermost catalytic reaction zone 130 or at top 124. Each catalytic reaction zone 130 is between reaction zone lower bound 132 and reaction zone upper bound 134. Moving catalyst bed reactor 120 in Figure 1 has three catalytic reaction zones. Each catalytic reaction zone 130 overlaps one another vertically due to the physical configuration of internal perforated collection funnel 128.

Moving catalyst bed reactor 120 forms hydrocarbon product from the interaction of introduced hydrocarbon material with direct cracking catalyst 126. Moving catalyst bed reactor 120 accepts the introduction of moving bed reactor feed 102 proximate to bottom 122. Once introduced into moving catalyst bed reactor 120, the combination of hydrocarbon material, steam and other optional materials, flows in a generally upwards direction (represented by solid arrow 136) from the location proximate to bottom 122 towards top 124. The inlet fluid flow, the pressure differential across moving catalyst bed reactor 120, the density of the various introduced and processing fluids and the temperature gradient of moving catalyst bed reactor 120 all contribute to the general upwards movement of hydrocarbon material and hydrocarbon product. Hydrocarbon product passes from moving catalyst bed reactor 120 through product hydrocarbons 116 at top 124.

Moving catalyst bed reactor 120 is operable to accept the introduction of solid direct cracking catalyst 126 through catalyst injection lines 140 proximate to the top of each catalytic reaction zone 130. As direct cracking catalyst 126 enters moving catalyst bed reactor 120, it disperses into each catalytic reaction zone 130, which contains hydrocarbon material, hydrocarbon cracking intermediates and hydrocarbon product, and active and spent cracking catalyst. Direct cracking catalyst 126 in each catalytic reaction zone 130 eventually moves downward under the influence of gravity, settling on top of internal perforated collection funnel 128 or other direct cracking catalyst 126, forming moving catalyst bed 142. Catalyst removal lines 144 remove a portion of direct cracking catalyst 126 from moving catalyst bed 142, causing the contents of moving catalyst bed 142 to continually move and shift in a downward direction where the shape and size of moving catalyst bed 142 remains stable.

Moving catalyst bed reactor 120 maintains an internal temperature gradient (represented by striped arrow 146). The temperature of both the fluids and solids increase as a function of height (from bottom 122 to top 124) in moving catalyst bed reactor 120. Temperature regulation systems (not shown) maintain a linear temperature gradient between bottom 122 and top 124 along the operative length of moving catalyst bed reactor 120.

Upon introduction, hydrocarbon material, oxygen, hydrogen and other reactive species, including heterorganic and organometallic compounds, intimately contact and intermingle with direct cracking catalyst 126. The reactants and reactive species contact direct cracking catalyst 126 when it is both freely moving around each catalytic reaction zone 130 as well as in moving catalyst bed 142. In moving catalyst bed 142, the upwardly mobile hydrocarbon material moves in, around and through the spaces formed between individual particles of direct cracking catalyst 126.

After initial introduction proximate to bottom 122, hydrocarbon material and reaction products traverse upwards through moving catalyst bed reactor 120 (in the general direction of solid arrow 136), continuing to intimately interact with direct cracking catalyst 126. As the reactants and products continue along the operative length of moving catalyst bed reactor 120, the temperature inside moving catalyst bed reactor 120 increases with the operative length (along striped arrow 146). As the internal temperature increases, both the direct cracking catalyst and the various species of hydrocarbons in the hydrocarbon material become more active. With increasing temperature and activity, direct cracking reactions occur with smaller and smaller hydrocarbons.

The trend of creating greater amounts of smaller and smaller hydrocarbon compounds from the hydrocarbon material using direct catalyst cracking continues until hydrocarbon product passes from moving catalyst bed reactor 120 through product hydrocarbons 116. The hydrocarbon product includes various amounts of hydrogen, sour gases, fuel gas, LPG, olefins, paraffins and aromatics.

Reactor recycle stream 148 feeds a portion of product hydrocarbons 116 back into bottom 122 to support fluid flow and provide feedstock for additional cracked products.

Direct catalytic cracking system 100 also includes catalyst regenerator 150. Catalyst regenerator 150 uses oxygen from heated air 114 to purge and regenerate direct cracking catalyst 126. Catalyst regenerator 150 couples to moving catalyst bed reactor 120 through catalyst injection line 140 and catalyst removal line 144. Direct catalytic cracking system 100 introduces direct cracking catalyst 126 from moving catalyst bed reactor 120 into catalyst regenerator 150 through catalyst removal line 144. Catalyst regenerator 150 passes oxidized coke reaction products through catalyst residue off-gas 118. Direct catalytic cracking system 100 introduces direct cracking catalyst 126, now regenerated, into moving catalyst bed reactor 120 through catalyst injection line 140.

Direct catalytic cracking system 100 also receives make-up catalyst through fresh direct cracking catalyst feed 112. Fresh direct cracking catalyst feed 112 couples to catalyst injection line 140 to introduce new direct cracking catalyst 126.

### Figure 2

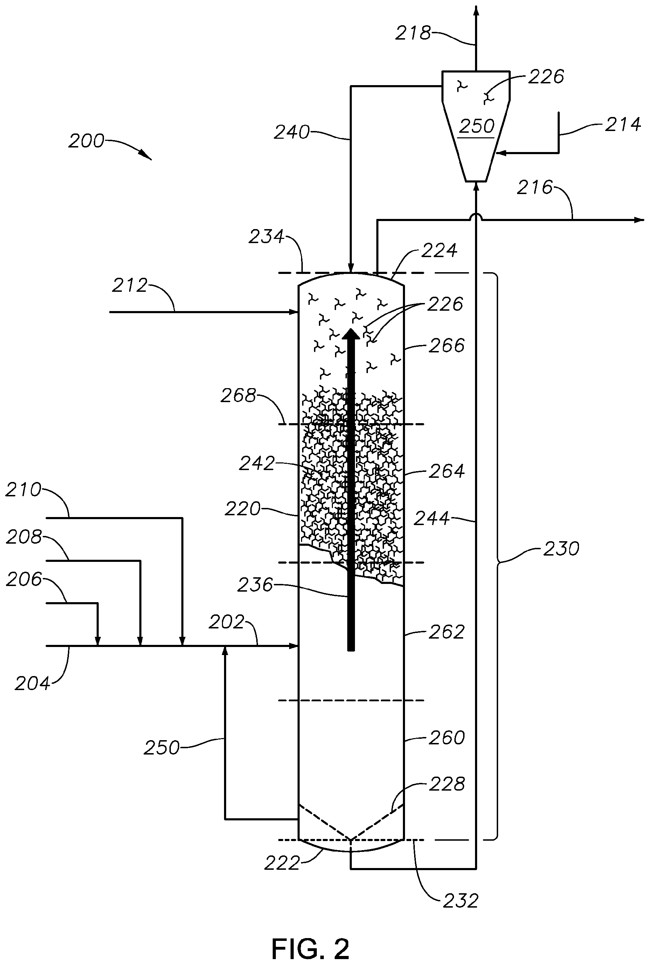

Figure 2 is a general flow schematic of another direct catalytic cracking system. Direct catalytic cracking system 200 has many similar aspects to direct catalytic cracking system 200 of Figure 1. Similar streams and equipment between Figures 1 and 2 share similar numbers. Figure 2 and its description facilitate a better understanding of direct catalytic cracking systems and processes. In no way should Figure 2 limit or define the scope of the invention.

Direct catalytic cracking system 200 has several differences with respect to direct catalytic cracking system 100. Moving catalyst bed reactor 220 only has one catalytic reaction zone 230. Moving bed reactor feed 202 and fresh direct cracking catalyst feed 212 both directly introduce feeds into catalytic reaction zone 230. The position of moving bed reactor feed 202 along moving catalyst bed reactor 220 is not proximate to bottom 222.

Direct catalytic cracking system 200 also has a different recycle configuration than direct catalytic cracking system 100. In Figure 1, reactor recycle stream 148 splits from product hydrocarbons 116 and recycles material that has passed through top 124. In Figure 2, the configuration of moving catalyst bed reactor 220 takes a bottoms material recycle where bottoms recycle 250 directs a portion of fluid flow back into moving bed reactor feed 202 from proximate to bottom 222. The bottoms material recycles to a point higher up moving catalyst bed reactor 220 where the temperature is greater.

Moving catalyst bed reactor 220, besides the aforementioned process flow differences, also has a different temperature management and control arrangement. Moving catalyst bed reactor 220 has more than one internal fixed temperature zone. The four different internal temperature zones of moving catalyst bed reactor 220 are temperature zone 1 (260), temperature zone 2 (262), temperature zone 3 (264), and temperature zone 4 (266). Dashed line 268 demarcates the upper and lower bounds of each internal fixed temperature zone. Direct catalytic cracking system 200 maintains the temperature in each internal fixed temperature zone at a constant temperature throughout the temperature zone.

To facilitate the desirable catalytic cracking reactions in moving catalyst bed reactor 220, the temperatures in each internal fixed temperature zone are successively greater in relationship to distance from the location of introduction of the moving bed reactor feed 202. For example, direct catalytic cracking system 200 maintains the temperature in temperature zone 1 (260) at a lower temperature relative to the temperature in temperature zone 2 (262). The temperature maintained in temperature zone 4 (266) is the highest of the four given temperature zones; the temperature maintained in temperature zone 1 (260) is the lowest of the four given temperature zones.

### Moving bed reactor feed

The direct catalytic cracking system includes a moving bed reactor feed. The moving bed reactor feed includes hydrocarbon material and steam. Optionally, the moving bed reactor feed includes hydrogen, oxygen or both. The moving bed reactor feed can also incorporate a recycle stream from another part of the direct catalytic cracking system to improve conversion efficiency.

The moving bed reactor feed is a vaporized, gaseous or a saturated vapor; a liquid or a dual-phase material. An embodiment of the method includes introducing the moving bed reactor feed as a vapor. The steam co-feed or a pre-heater provides thermal energy to vaporize that portion of the moving bed reactor feed that is not already in a vapor state.

An embodiment of the method includes introducing a moving bed reactor feed such that the liquid hourly space velocity (LHSV) of the hydrocarbons in the moving catalyst bed reactor is in a range of from about 0.1 hr⁻¹ to about 50 hr⁻¹.

### Hydrocarbon material

The hydrocarbon material includes unfractionated and undistilled crude oil. The hydrocarbon material can include unfractionated and undistilled liquid condensate or natural gas.

Crude oil is a hydrocarbon-based liquid (at atmospheric temperatures and pressures on the surface) that issues from oil recovery systems. The distillation or fractionation of crude oil is not required. Unfractionated and undistilled crude oil usually contains some amount of suspended solids, including asphaltenes and tars.

Natural gas sources usually produce a liquid condensate contemporaneously with gas production. Unfractionated and undistilled liquid condensate, which is a hydrocarbon liquid at surface conditions, contains a range of light hydrocarbons, including BTEX aromatics, cycloparaffins and long-chain paraffins. Liquid condensate has similar hydrocarbons as the lightest liquid hydrocarbon fraction present in crude oil (C5 to about C20 range, including both paraffins and aromatics).

Natural and associated gases (that is, natural gases) are gaseous hydrocarbon material at surface conditions. Natural and associated gases contain hydrogen, methane, ethane, propane butanes, pentanes and hexanes. The natural and associated gases can also contain other non-hydrocarbon gases, including nitrogen, noble gases, oxygen, carbon oxides, water and hydrogen sulfide.

The term "hydrocarbon" and variants includes other materials besides those bearing only hydrogen and carbon atoms. An untreated and undistilled hydrocarbon material contains heterorganic compounds that include oxygen, sulfur and nitrogen, including alkane and cycloalkane sulfides, mercaptans, disulfides, polysulfides and thiophenes. Hydrocarbons also include organometallic compounds including vanadium, copper and nickel. Hydrocarbon material can contain up to several percent by weight of heterorganic compounds based upon its source. In addition, the hydrocarbon material can also include inorganic ions and salts.

An embodiment of the method includes introducing moving bed reactor feed containing hydrocarbon material having previously processed, distilled or fractionated hydrocarbons, which includes atmospheric pressure boiling point crude oil fractions. Examples of distilled or fractionated hydrocarbon materials useful to supplement the hydrocarbon material includes naphtha, kerosene, diesel gas oil, vacuum-based distillates, paraffin oils and fractions containing some amount of asphaltenes and maltenes.

An embodiment of the method includes introducing moving bed reactor feed containing hydrocarbon material having waste and recovered fluid streams from other portions of the refinery, petrochemical processes and consumer waste. Gaseous waste streams include off-gases, waste gases and tails gases. Liquid waste streams include contaminated and recycled heavy oils and waste streams from other refinery or petrochemical processes. Hydrocarbon material also includes vegetable and animal oils, including "fast-food" cooking oils, vehicle oils, spent hydrocarbon solvents and heat transfer fluids. The direct catalytic cracking system exploits the olefins and aromatic materials present in the waste materials and creates additional aromatic and olefinic materials. The waste material can be relatively inexpensive to acquire and blend with hydrocarbon material.

### Steam

The steam can be wet, dry or superheated steam. An embodiment of the method includes introducing a moving bed reactor feed containing superheated steam.

The moving bed reactor feed includes steam to dilute the hydrocarbon molecules in the feed and lower their hydrocarbon partial pressure. Lower hydrocarbon partial pressure enhances the formation of olefins and aromatics in the moving catalyst bed reactor. Steam reduces the rate of carbon deposition on the catalyst or coke formation. Steam acts as a source of *in-situ* hydrogen, which is useful for hydrotreating or hydrocracking the hydrocarbon material in the moving bed reactor feed.

Steam provides heat to the moving bed reactor feed. An embodiment of the method includes introducing steam having a temperature in a range of from about 150 °C to about 800 °C. The heat from the steam can partially vaporize the moving bed reactor feed before it enters the moving catalyst bed reactor. Depending on the content of the hydrocarbon material, some of the heaviest hydrocarbon species can also thermally crack, although this is not an objective.

An embodiment of the method includes introducing a moving bed reactor feed having a weight ratio of steam to hydrocarbon material in a range of from about 0.1 to about 2.

### Oxygen

An embodiment of the method includes introducing a moving bed reactor feed containing oxygen. Oxygen, at the operating temperatures of the moving catalyst bed reactor, forms radical ions that are operable to react with and break down large aromatic and aliphatic hydrocarbon molecules into smaller ones. In addition, oxygen can react with carbon on the surface of the direct cracking catalyst such that carbon oxides form, removing the solid from the surface of the catalyst. Oxygen can also form metal oxides with organometallic impurities, releasing them from the metal/organic compounds and prevent them from poisoning the direct cracking catalyst.

The introduced oxygen is in the form of pure oxygen or as a mixture; however, air or "enriched air" is not used. Avoiding air reduces the amount of byproduct NOx compounds formed in the process. An embodiment of the method includes introducing a moving bed reactor feed containing oxygen having a purity of at least 90% oxygen on a dry molar basis. An embodiment of the method includes introducing a moving bed reactor feed having a weight ratio of oxygen to hydrocarbon material in a range of from about 0.01 to about 1.

### Hydrogen

An embodiment of the method includes introducing a moving bed reactor feed containing hydrogen. Hydrogen facilitates the removal of certain contaminants, including sulfur and nitrogen heterorganic compounds present in the hydrocarbon material.

The introduced hydrogen is pure or a mixture. As a blend, the hydrogen can be in a hydrogen-rich stream containing either inorganic or organic compounds, including carbon oxides or methane. Purge streams from hydrogen-generating systems are useful hydrogen sources. An embodiment of the method includes introducing a moving bed reactor feed containing hydrogen having a purity of at least 90% hydrogen on a dry molar basis. An embodiment of the method includes introducing a moving bed reactor feed having a weight ratio of hydrogen to hydrocarbon material in a range of from about 0.01 to about 1.

### Recycled hydrocarbon material

An embodiment of the method includes introducing a moving bed reactor feed containing recyclable hydrocarbon material. Recycled hydrocarbon material contains recyclable hydrocarbons suitable for conversion into olefins and aromatics.

The hydrocarbon product stream is a source for recyclable hydrocarbon material. The product stream can contain paraffins and cycloparaffins, olefins and aromatics that are a source for smaller, more desirable paraffins, olefins and aromatics with additional processing in the moving catalyst bed reactor. An embodiment of the direct catalytic cracking system is operable to introduce a portion of the hydrocarbon product to the moving bed reactor as part of the moving bed reactor feed.

The moving catalyst bed reactor itself is a source for recyclable hydrocarbon material. A recycle stream can pass hydrocarbons from one portion of the moving catalyst bed reactor to the moving bed reactor feed. An embodiment of the direct catalytic cracking system includes where the moving catalyst bed reactor is further operable to pass hydrocarbons from a first portion of the moving catalyst bed reactor to a second portion of the moving catalyst bed reactor, where the second portion is more proximate to the position where the moving bed reactor feed is introduced than the first position. In an embodiment of the direct catalytic cracking system, the first portion has a first temperature and the second portion has a second temperature, and where the first temperature is maintained at a greater temperature than the second temperature. The continuous recycle of hydrocarbons to and from different portions of the moving catalyst bed reactor optimizes the cracking of hydrocarbons in the moving catalyst bed reactor, especially in embodiments of the direct catalyst cracking system having moving catalyst bed reactors with multiple catalytic reaction zones. Continuous reactor recycle can also produce "recycle until oblivion" for the heaviest of the introduced hydrocarbon material, where all introduced material eventually converts into hydrocarbon product, hydrogen and coke.

The catalyst regenerator system is a source for recyclable hydrocarbon material. Extraction methods for capturing recyclable hydrocarbons from the surface of the spent direct cracking catalyst include hydrocarbon solvents or exposure to sub-atmospheric pressure.

An embodiment of the method includes introducing a moving bed reactor feed having a weight ratio of recyclable hydrocarbon material to hydrocarbon material in a range of from about 0.1 to about 0.75. An embodiment of the method includes introducing a moving bed reactor feed having a weight ratio of product hydrocarbons to hydrocarbon material in a range of from about 0.2 to about 0.75.

### Direct cracking catalyst

The direct cracking catalyst is operable to convert larger paraffins and aromatics into smaller paraffins, olefins and aromatics. The direct cracking catalyst fosters cracking of the hydrocarbons in the hydrocarbon material, which includes fatty acids and waxy paraffins, alkyl aromatics, naphthenes and polynuclear aromatic hydrocarbons. The hydrocarbons crack into smaller product hydrocarbon compounds and hydrogen. As the smaller hydrocarbons traverse the operating length of the reactor, they crack into even smaller paraffins, olefins and aromatics.

An embodiment of the method includes using a supported heterogeneous material as a direct cracking catalyst. Supported heterogeneous material has a solid substrate that supports active catalytic material. An embodiment of the method includes using a solid homogeneous material as a direct cracking catalyst. A solid homogeneous material is where the entire material is useful as a direct cracking agent. The direct cracking catalyst is not buoyant in fluids but is capable of temporary buoyancy through continuous entrainment. Configurations of the direct cracking catalyst prevent clumping or mechanical interlocking, which avoids the formation of "clots" or "clumps" of solid material in the moving catalyst bed reactor.

The direct cracking catalyst typically includes a mixture of fresh and regenerated catalyst. Regenerated catalyst is direct cracking catalyst that has been previously used (that is, "spent") that has been treated to remove coke and metal impurities. Regenerated direct cracking catalyst is as effective as fresh direct cracking catalyst. The catalyst fed to the moving catalyst bed reactor and in the moving catalyst bed reactor is mostly regenerated catalyst. A catalyst "make up" line supplies fresh catalyst to the direct catalyst cracking system to replace lost and deactivated catalyst.

Preheating the direct cracking catalyst prior to introduction into the moving catalyst bed reactor provides latent heat directly into the interior of the moving catalyst bed reactor and supports the cracking reactions.

The catalyst feed into the moving catalyst bed reactor can take the form of a single or multiple streams. In the direct catalytic cracking system at least one catalyst feed stream introduces direct cracking catalyst into each catalytic reaction zone. An embodiment of a method of using a direct catalytic cracking system includes introducing fresh (that is, virgin) direct cracking catalyst exclusively into a first catalytic reaction zone of a moving catalyst bed reactor with more than one catalytic reaction zones.

An embodiment of the direct catalytic cracking system includes a moving catalyst bed reactor with a single catalytic reaction zone having more than one catalyst feed stream, where each catalyst feed stream is positioned in different locations along the operable length of the moving catalyst bed reactor. Such an embodiment can improve direct catalytic cracking activity at those locations along the operative length of the reactor.

### Hydrocarbon product

The hydrocarbon product passes from the moving catalyst bed reactor as a mixture of paraffinic, olefinic and aromatic hydrocarbons, especially "light" olefins, including ethylene and propylene, and BTEX aromatics, including mixed xylenes and especially para-xylene. In addition, hydrogen also passes as a part of the hydrocarbon product. Downstream separations systems from the direct catalytic cracking system split the hydrogen, olefins and BTEX aromatics for further processing into useful petrochemical feedstocks. Cooling the vaporized hydrocarbon product stream in a timely manner prevents additional thermal cracking, polymerization or other side reactions of the olefins and preserves them for petrochemical use.

An embodiment of the method includes operating the moving catalyst bed reactor such that the weight ratio of light olefins to C6-8 aromatics in the hydrocarbon product is in a range of from about 2 to about 20. An embodiment of the method includes operating the moving catalyst bed reactor such that the weight ratio of ethylene to propylene in the hydrocarbon product is in a range of from about 1:5 to about 5: 1.

### Spent direct cracking catalyst

The moving catalyst bed reactor passes spent direct cracking catalyst. The direct cracking catalyst is a deactivated form of the direct cracking catalyst. Solid and liquid hydrocarbon and residuum of the cracking reactions, including coke, cover the surface of the spent direct cracking catalyst. Spent direct cracking catalyst can also contain non-catalytic metals introduced with the undistilled and unfractioned hydrocarbon material.

The direct catalytic cracking system passes the spent direct cracking catalyst from the moving catalyst bed reactor as solid or gelatinous-like slurry using well-known conveyance means, including screw drives and nitrogen pneumatic blowers, to the catalyst regeneration system.

### Moving catalyst bed reactor

The direct catalytic cracking system includes the moving catalyst bed reactor. The moving catalyst bed reactor is operable to convert hydrocarbon material into useful olefins and aromatics using the direct cracking catalyst. The moving catalyst bed reactor is operable to both receive the moving bed reactor feed and to pass the hydrocarbon product. Introduced hydrocarbon material moves from where it is introduced into the reactor to where the hydrocarbon product passes from the reactor.

The "operative length" of the moving catalyst bed reactor is the length aligned to the flow axis of the hydrocarbon fluids moving through one or more catalytic reaction zones. A line between the upstream-most point of a first catalytic reaction zone and the downstream-most point of a second catalytic reaction zone that is aligned with the general flow direction of the hydrocarbon material through the moving catalyst bed reactor gives reactor operative length. It is important to note that reactor operative length is not necessarily the same value as cumulative catalytic reaction zone operative length for a moving catalytic bed reactor. The operative lengths of individual catalytic reaction zones can overlap due to catalytic reaction zone configuration.

Along the operative length of the moving catalyst bed reactor, the hydrocarbon material passes through at least one catalytic reaction zone that contains direct cracking catalyst. The moving catalyst bed reactor is operable to permit the intermingling of hydrocarbons with the direct cracking catalyst in the moving catalyst bed along at least a portion of the operative length of the moving catalyst bed reactor.

The positions along the moving catalyst bed reactor where the moving bed reactor feed, the direct cracking catalyst feed and the recyclable hydrocarbon material introduction occurs can vary. As well, the points of extraction for spent direct cracking catalyst, hydrocarbon product and the recyclable hydrocarbon material streams can vary. Any or all of the feed and product streams can have single or multiple points of entry to and removal from the moving catalyst bed reactor. The number and position of catalytic reaction zones in the moving catalyst bed reactor influences the location of catalyst feed and removal lines as each catalytic reaction zone has at least one catalyst feed line and at least one spent catalyst removal line.

### Catalytic reaction zone

The moving catalyst bed reactor includes at least one catalytic reaction zone. The catalytic reaction zone is a portion of the interior of the moving catalyst bed reactor where catalytic hydrocarbon cracking reactions occur in the presence of the direct cracking catalyst. Aggregations of direct cracking catalyst, especially the moving catalyst bed, promote the cracking reactions.

The moving catalyst bed reactor includes at least one catalyst reaction zone, where each catalytic reaction zone is operable to receive the direct cracking catalyst, to contain an amount of the direct cracking catalyst in the form of a moving catalyst bed and to pass a spent direct cracking catalyst such that it egresses from the reactor. The status of the direct cracking catalyst in the catalyst bed varies from fresh to spent. Where the moving catalyst bed reactor has multiple catalytic reaction zones, each catalytic reaction zone maintains the direct cracking catalyst in its zone separate from the direct cracking catalyst in an adjacent zone.

The catalytic reaction zone, especially in moving catalyst bed reactors with multiple catalytic reaction zones, is operable to permit fluid to traverse through the moving catalyst bed reactor with minimal structural hindrance. The moving catalyst bed in each catalytic reaction zone provides some flow hindrance that promotes direct contact with the direct cracking catalyst, fostering hydrocarbon cracking reactions.

In moving catalyst bed reactors having multiple catalytic reaction zones, adjacent catalytic reaction zones fluidly couple with one another through at least one intermediary solids separation device. A solids separations device provides minimal fluid flow resistance but is a barrier to solid particles, especially to the direct cracking catalyst. Mesh screens, perforated plates and fluid filters are examples of useful solids separations devices. The position of the solids separations device permits the gathering of spent direct cracking catalyst inside the catalytic reaction zone and the formation and maintenance of the moving catalyst bed.

The operative length of the catalytic reaction zone is the differential length of the catalytic reaction zone from a first operative end to a second operative end, as measured along a line aligned with the hydrocarbon fluid flow through the catalytic reaction zone. The two operative ends are generally on opposing portions of the catalytic reaction zone (for example, top and bottom ends; upstream and downstream; proximal and distal) that reflect the furthest points in the catalytic reaction zone where direct cracking catalyst can access hydrocarbons and facilitate cracking.

For example, a catalytic reaction zone in a vertically oriented moving catalyst bed reactor can have bounds along the hydrocarbon flow axis of a rounded vessel top and a downwardly sloped catalyst collection plate. The operative length of the aforementioned catalytic reaction zone is the distance between the highest point (along the top-most portion of the internal surface of the rounded top) and the lowest point (along the bottom-most point of the catalyst plate surface where catalyst can still contact the hydrocarbon fluid) as measured along the length of the axis aligned with the hydrocarbon fluid flow, which is in a vertical direction.

In the moving catalyst bed reactor with a single catalytic reaction zone, the operative length of the catalytic reaction zone is usually less than the structural internal length of the reactor. The operative length is likely less than vessel internal length due to internal catalyst collection, management and distribution structures, including nozzles, funnels and catalyst bed support plates. These structures limit the movement of the solid catalyst.

An embodiment of the moving catalyst bed reactor includes the operative length and position of each catalytic reaction zone within the moving catalyst bed reactor is associated with the position of each internal fixed temperature zone.

### Catalyst movement in a catalytic reaction zone

The direct catalyst cracking system introduces direct cracking catalyst into the catalytic reaction zone of the moving catalyst bed reactor either in a continuous, semi-continuous or periodic manner. The feed rate of direct cracking catalyst into the moving catalyst bed reactor is coordinated with the withdrawal rate of spent direct cracking catalyst such that the moving catalyst bed length remains relatively steady. In doing so, the amount of direct cracking catalyst present in the reactor at any given point in time is essentially constant.

Once introduced into the moving catalyst bed reactor, each direct cracking catalyst particle migrates into and through the hydrocarbons, which includes pre- and post-cracked hydrocarbons, present in the reactor. In time, an individual direct cracking catalyst particle migrates through the catalytic reaction zone into which it is introduced such that it incorporates into and becomes part of the moving catalyst bed in the zone. As the direct catalytic cracking system continually removes spent direct cracking catalyst from and introduces direct cracking catalyst into the moving catalyst bed reactor, each individual catalyst particle continues to migrate as part of the moving bed towards the position in the catalytic reaction zone where it is removed for recycling.

An embodiment of the direct catalytic cracking system includes a moving catalyst bed reactor that exploits the effects of gravity on dense, solid objects flowing through less-dense fluids. In such a moving catalyst bed reactor, the introduction of the direct cracking catalyst occurs at an elevated position in the catalytic reaction zone. The solid direct cracking catalyst generally migrates in a downward direction while intimately intermingling with upward flowing hydrocarbons. Upon reaching a lower portion of the catalytic reaction zone, the direct cracking catalyst settles onto solids restraining plates, vessel walls or into the moving catalyst bed until it passes from the reactor. While still in the catalytic reaction zone, the direct cracking catalyst continues to intermingle with the generally upward flowing hydrocarbons. An embodiment of the method of using the direct catalytic cracking system includes operating the moving catalyst bed reactor such that the average residence time for the direct cracking catalyst in the moving catalyst bed reactor is in a range of from about 0.5 to about 5 hours.

The moving and shifting of direct cracking catalyst in the moving catalyst bed from the coordinated addition and removal of direct cracking catalyst causes flow channels, spaces and gaps to continually form, alter and collapse. This dynamic fluid flow environment creates continuous collisions between the solid direct cracking catalyst and the hydrocarbons flowing around it. If catalytically active and at the appropriate operating temperature range, the direct cracking catalyst converts the larger hydrocarbons into smaller hydrocarbons.

The length of the moving catalyst bed in any catalytic reaction zone is no greater than the operative catalytic reaction zone length. The moving catalyst bed length is the differential length of the moving catalyst bed from the upstream portion to the downstream portion (or from top to bottom; proximate to distal) as measured along a line aligned with the hydrocarbon fluid flow through the catalytic reaction zone.

### Moving catalyst bed reactor internal temperature profile

The direct catalytic cracking system is operable to maintain the moving catalyst bed reactor such that a temperature differential exists between the point of introduction of the moving bed reactor feed and the position where hydrocarbon product passes. The temperature differential along the operative length of the moving catalyst bed reactor promotes direct cracking along the entire operable length of the reactor. The increasing internal temperature profile as a function of operable length, by passing through different fixed temperature zones, limits the formation of coke on the direct cracking catalyst at all points through the moving catalyst bed reactor.

The temperature inside the moving catalyst bed reactor helps to promote the cracking reactions in the presence of the direct cracking catalyst. Hydrocarbon cracking is endothermic by nature, and the heat provided from both external sources and the moving bed reactor feed fosters the continuous cracking of hydrocarbons all along the operative length of the reactor.

At a given temperature, hydrocarbons with a greater carbon count tend to react before hydrocarbons with a smaller carbon count. For example, n-heptane as a hydrocarbon constituent of the introduced hydrocarbon material may not crack upon initial introduction into the moving catalyst bed reactor. The presence of larger molecules, for example, paraffins, alkyl aromatics and unsaturated hydrocarbons in the C8-30 range and lower operating temperatures near the point of introduction, foster an environment where a portion of or only C8+ compounds catalytically crack into smaller compounds.

In reference to the relative use of the words "smaller", such as "smaller olefins" or "smaller aromatics", and its opposite "larger", "smaller" and "larger" indicates a comparison of the number of carbon-carbon bonds in an original reactant compound versus one or more product compound(s) or in comparison between a reactant and its products. The direct catalytic cracking reaction breaks down certain carbon-carbon bonds of a reactant hydrocarbon molecule and forms resultant hydrocarbon molecules, each product molecule having fewer carbon atoms than the reactant compound but additively having the same number of carbon atoms between reactants and products. For example, the direct catalytic cracking of normal dodecane, a C12, can result in the formation of normal butane, a C4, and 1-octene, a C8, as products. Although the total number of carbon atoms has not changed, both n-butane and 1-octene are "smaller" than n-dodecane because each product individually bears fewer carbon atoms than its reactant source. To continue the example, the product 1-octene is "larger" than n-butane because it has a greater number of carbon atoms than n-butene does.

As hydrocarbons traverse along the operating length of the moving catalyst bed reactor, the temperature increases inside the reactor. The increasing internal temperatures gradually favor the cracking of smaller and smaller hydrocarbons until, using the previous example, conditions favor the cracking of n-heptane.

At the operating conditions in the moving catalyst bed reactor, the catalyst promotes direct catalytic cracking of the hydrocarbon materials previously contained in the moving bed reactor feed, including fatty acids and waxy paraffins, alkyl aromatics, naphthenes and polynuclear aromatic hydrocarbons. The hydrocarbons in the hydrocarbon material crack into smaller hydrocarbons comprising paraffins, olefins and aromatics. Hydrogen is also a product. These smaller hydrocarbons can also crack into even smaller paraffins, olefins, and aromatics elsewhere in the moving catalyst bed reactor, eventually forming the product hydrocarbon.

### Internal temperature profile

The moving catalyst bed reactor is operable to maintain an internal temperature profile for the moving catalyst bed reactor along its operative length. Along the operative length of the reactor, the internal temperature is different at different positions, especially as compared between the introduction position of the moving bed reactor feed and the passing point of the hydrocarbon product. The internal reactor temperatures at various points along the operative length of the reactor characterize the internal temperature profile.

An embodiment of the method of using a direct catalytic cracking system includes operating the moving catalyst bed reactor such that the internal temperature profile is maintained within a range of from about 200 °C to about 1000 °C. An embodiment of the method includes operating such that the internal temperature profile is maintained within a range of from about 300 °C to about 850 °C. An embodiment of the method includes operating such that the internal temperature profile is maintained within a range of from about 350 °C to about 750 °C.

### Internal fixed temperature zones

The direct catalyst cracking system includes a moving bed reactor that is operable to maintain an internal temperature profile with more than one fixed temperature zone along the operating length of the moving catalyst bed reactor. The method of using a direct catalytic cracking system includes operating the moving catalyst bed reactor such that the internal temperature profile is maintained for the operative length of the reactor more as more than one fixed temperature zone along the operating length of the moving catalyst bed reactor. 2. A J first fixed temperature zone having a first temperature is positioned proximate to the position of introduction for the moving bed reactor feed and a second fixed temperature zone having a second temperature is positioned proximate to the position of passing for the product hydrocarbon. The second temperature is greater than the first temperature. The second temperature is the highest temperature and the first temperature is the lowest temperature along the operating length of the reactor.

The direct catalyst cracking system maintains within the moving catalyst bed reactor at least a portion of an internal fixed volume along the operating length such that the temperature within the volume is effectively isothermic in nature, even with the movement of hydrocarbons and direct cracking catalyst through the temperature zone. The direct catalyst cracking system includes having a moving catalyst bed reactor that is operable to maintain more than one internal fixed temperature zone within the moving catalyst bed reactor, where the temperature maintained in a downstream temperature zone is greater than the temperature maintained in an adjacent upstream temperature zone, where upstream and downstream are defined relative to the introduction point of the moving bed reactor feed. An embodiment of a method of using the direct catalyst cracking system includes maintaining a temperature different between adjacent temperature zones in a range of from about 10 °C to about 100 °C. Maintaining fixed temperature zones can generally regulate the location where certain types of hydrocarbons crack along the operative length of the moving catalyst bed reactor.

The direct catalyst cracking system includes a moving catalyst bed reactor where each catalytic reaction zone within the reactor is associated with a fixed temperature zone. As such, a first catalytic reaction zone pairs with a first temperature zone, a second catalytic reaction zone has a second temperature zone and so on. Cracking reaction management is relatively easier and coke formation more controlled while using internal fixed temperature zones to regulate moving catalyst bed internal temperature. In a hypothetical catalytic moving bed reactor with matched temperature and catalyst zones, the direct catalytic cracking of the hydrocarbons progresses to certain equilibrium and no further when the temperature is steady. When the hydrocarbon flows into a different, higher temperature zone with a different moving catalyst bed, the hydrocarbon cracking reactions begin and continue until a new equilibrium forms. In addition, the catalysts, made of solid materials, usually retain adsorbed heat, helping to maintain the temperature in the internal fixed temperature zone.

An embodiment of the direct catalyst cracking system includes a moving catalyst bed reactor where each fixed temperature zone has a length as measured along the operating length of the moving catalyst bed reactor in a range of from about 1 meter to about 50 meters.

### Catalyst regeneration systems

The direct catalytic cracking system includes the catalyst regeneration system. The catalyst regenerator couples to the moving catalyst bed reactor such that the direct catalytic cracking system is operable to introduce direct cracking catalyst from the catalyst regeneration system to the at least one catalytic reaction zone through catalyst feed lines. The catalyst regenerator couples to the moving catalyst bed reactor such that the direct catalytic cracking system is operable to introduce spent direct cracking catalyst from the at least one catalytic reaction zone to the catalyst regeneration system.

The catalyst regeneration system is operable to convert spent direct cracking catalyst into regenerated direct cracking catalyst. Direct catalytic cracking of hydrocarbons, even at lower temperatures, eventually collects coke on the surface of the direct cracking catalyst. Coke and other carbon residues contaminate the surface of the direct cracking catalyst, clogging its surface pores and blocking the catalytically active surface. In addition, metals and non-metals can degrade the selectivity and efficiency of the direct cracking catalyst. Regenerated direct cracking catalyst is operable to crack hydrocarbons at moving catalyst bed reactor operating conditions with a similar operating effectiveness as that of new catalyst.

The catalyst regeneration system is operable to convert the spent catalyst to regenerated catalyst using a source of oxygen. Using air or oxygen at high temperatures is known to treat spent catalyst. Oxygen delivered at high temperatures oxidizes the coke on the surface of the direct cracking catalyst as well as any residual hydrocarbons present. The oxidation reaction produces carbon oxides. The direct catalytic cracking system routes the carbon oxides off-gas from the regeneration system. The oxidation reaction also converts any catalyst poisons and metals with the spent direct cracking catalyst into metal and non-metal oxides. These oxides either remain with the regenerated direct cracking catalyst and no longer act as functional inhibitors or are expelled with the off-gas

### Supporting equipment

Embodiments include many additional standard components or equipment that enables and makes operable the described apparatus, process, method and system. Examples of such standard equipment known to one of ordinary skill in the art includes heat exchanges, pumps, blowers, reboilers, steam generation, condensate handling, membranes, single and multi-stage compressors, separation and fractionation equipment, valves, switches, controllers and pressure-, temperature-, level- and flow-sensing devices.

Operation, control and performance of portions of or entire steps of a process or method can occur through human interaction, pre-programmed computer control and response systems, or combinations thereof.

## Claims

1. A method of converting undistilled and unfractionated hydrocarbon material into a hydrocarbon product using a direct catalytic cracking system, the method comprising the steps of: introducing a moving bed reactor feed into a moving catalyst bed reactor of the direct catalytic cracking system such that the moving bed reactor feed intermingles with a direct cracking catalyst contained in a catalytic reaction zone within the moving catalyst bed reactor, where the moving bed reactor feed comprises steam and an undistilled and unfractionated hydrocarbon material; introducing the direct cracking catalyst into the catalytic reaction zone of the moving catalyst bed reactor; and operating the direct catalytic cracking system such that an internal temperature profile is maintained along an operative length of the moving catalyst bed reactor, such that a moving catalyst bed comprising the direct cracking catalyst is maintained in the catalytic reaction zone, such that the hydrocarbon material converts in the presence of the direct cracking catalyst into a product hydrocarbon and the direct cracking catalyst converts into a spent direct cracking catalyst in the catalytic reaction zone, such that the spent direct cracking catalyst passes from the catalytic reaction zone to a catalyst regeneration system, and such that the hydrocarbon product is produced from the moving catalyst bed reactor, the hydrocarbon product comprising light olefins and C6-8 aromatics; where the direct catalytic cracking system includes the moving catalyst bed reactor and the catalyst regeneration system, and where the moving catalyst bed reactor has the catalyst reaction zone and is operable to maintain the internal temperature profile for the moving catalyst bed reactor along its operative length,
wherein the internal temperature profile is maintained as more than one fixed temperature zone between a position of introduction for the moving bed reactor feed and a position of passing for the hydrocarbon product, where a first fixed temperature zone having a first temperature is positioned proximate to the position of introduction and a second fixed temperature zone having a second temperature is positioned proximate to the position of passing, and where the second temperature is greater than the first temperature, wherein the temperature increases from the first temperature to the second temperature along the operative length.

2. The method of claim 1 where the weight ratio of steam to the hydrocarbon material is in a range of from 0.1 to 2.

3. The method of claim 1 or claim 2 where the moving bed reactor feed further comprises oxygen.

4. The method of any one of claims 1 to 3 where the moving bed reactor feed further comprises hydrogen.

5. The method of any one of claims 1 to 4 where the moving bed reactor feed further comprises recyclable hydrocarbon material, where the weight ratio of recyclable hydrocarbon material to hydrocarbon material is in a range of from 0.1 to 0.75; optionally where the recyclable hydrocarbon material comprises product hydrocarbon, and where the weight ratio of product hydrocarbon to hydrocarbon material is in a range of from 0.2 to 0.75.

6. A direct catalytic cracking system that is operable to convert an undistilled and unfractionated hydrocarbon material into a hydrocarbon product comprising a mixture of light olefins and C6-8 aromatics using a direct cracking catalyst, the direct catalytic cracking system comprising: a moving catalyst bed reactor that is operable to receive a moving bed reactor feed comprising the undistilled and unfractioned hydrocarbon material and to pass a hydrocarbon product comprising a mixture of light olefins and C6-8 aromatics, that has more than one catalytic reaction zones, where each catalytic reaction zone is operable to receive the direct cracking catalyst, to contain an amount of the direct cracking catalyst in the form of a moving catalyst bed and to pass a spent direct cracking catalyst such that it egresses from the moving catalyst bed reactor, that is operable to permit the intermingling of hydrocarbons with the direct cracking catalyst in the moving catalyst bed along at least a portion of the operative length of the moving catalyst bed reactor, and having means for maintaining an internal temperature profile for the moving catalyst bed reactor along its operative length; and a catalyst regeneration system that couples to the moving catalyst bed reactor such that the direct catalytic cracking system is operable to introduce the direct cracking catalyst from the catalyst regeneration system to each catalytic reaction zone and is operable to introduce the spent direct cracking catalyst from each catalytic reaction zone to the catalyst regeneration system, and that is operable to convert the introduced spent direct cracking catalyst into the direct cracking catalyst using a source of oxygen,
wherein the internal temperature profile is maintained as more than one fixed temperature zone between a position of introduction for the moving bed reactor feed and a position of passing for the hydrocarbon product, where a first fixed temperature zone having a first temperature is positioned proximate to the position of introduction and a second fixed temperature zone having a second temperature is positioned proximate to the position of passing,
wherein the apparatus is configured such that the second temperature is greater than the first temperature and the temperature increases from the first temperature to the second temperature along the operative length; and
wherein each catalytic reaction zone is associated with one of the fixed temperature zones and adjacent catalytic reaction zones fluidly couple with one another through at least one intermediary solids separation device.

7. The direct catalytic cracking system of claim 6 where the moving catalyst bed reactor is further operable to pass recyclable hydrocarbon material from a first portion of the moving catalyst bed reactor to a second portion of the moving catalyst bed reactor, where the second portion is more proximate to the position where the moving bed reactor feed is introduced than the first position; optionally where the first portion has a first temperature and the second portion has a second temperature, and where the first temperature is maintained at a greater temperature than the second temperature.

8. The direct catalytic cracking system of any one of claims 6 to 7 where the system is operable to introduce a portion of the hydrocarbon product to the moving bed reactor as part of the moving bed reactor feed.

## Patentansprüche

1. Verfahren zum Umwandeln von undestilliertem und unfraktioniertem Kohlenwasserstoffmaterial in ein Kohlenwasserstoffprodukt, unter Verwendung eines direkten katalytischen Cracking-Systems, wobei das Verfahren folgende Schritte umfasst: Einleiten eines Bewegtbettreaktoreintrags in einen Katalysator-Bewegtbettreaktor des direkten katalytischen Cracking-Systems dergestalt, dass der Bewegtbettreaktoreintrag sich mit einem Katalysator für direktes Cracking vermischt, welcher in einem Katalysator-Bewegtbettreaktor enthalten ist, wobei der Bewegtbettreaktoreintrag Dampf und undestilliertes und unfraktioniertes Kohlenwasserstoffmaterial umfasst; Einleiten des Katalysators für direktes Cracking in den katalytischen Reaktionsbereich des Katalysator-Bewegtbettreaktors; und Betreiben des direkten katalytischen Cracking-Systems dergestalt, dass ein internes Temperaturprofil entlang einer operativen Länge des Katalysator-Bewegtbettreaktors dergestalt gehalten wird, dass ein Katalysator-Bewegtbett, welches den Katalysator für direktes Cracking umfasst, in dem katalytischen Reaktionsbereich dergestalt gehalten wird, dass sich das Kohlenwasserstoffmaterial im Beisein des Katalysators für direktes Cracking in einen Produkt-Kohlenwasserstoff umwandelt, und der Katalysator für direktes Cracking sich in einen verbrauchten Katalysator für direktes Cracking in dem katalytischen Reaktionsbereich umwandelt, dergestalt, dass der verbrauchte Katalysator für direktes Cracking aus dem katalytischen Reaktionsbereich in ein Katalysator-Regenerationssystem passiert, dergestalt, dass das KohlenwasserstoffProdukt aus dem Katalysator-Bewegtbettreaktor heraus produziert wird, wobei das Kohlenwasserstoffprodukt leichte Olefine und C6-8 Aromaten umfasst; wobei das direkte katalytische Cracking System den Katalysator-Bewegtbettreaktor und das Katalysator-Regenerationssystem einschließt und wobei der Katalysator-Bewegtbettreaktor den Katalysator-Reaktionsbereich aufweist und bedienbar ist, um das interne Temperaturprofil für den Katalysator-Bewegtbettreaktor über dessen operative Länge beizubehalten,
wobei das interne Temperaturprofil als mehr als ein fester Temperaturbereich zwischen einer Einleitungsposition für den Bewegtbettreaktoreintrag und einer Position für den Transport des Kohlenwasserstoffprodukts gehalten wird, wobei ein erster Festtemperaturbereich, welcher eine erste Temperatur aufweist, unmittelbar an der Einleitungsposition positioniert ist, und ein zweiter Festtemperaturbereich, welcher eine zweite Temperatur aufweist, unmittelbar an der Position für den Transport positioniert ist, und wobei die zweite Temperatur größer als die erste Temperatur ist, wobei sich die Temperatur von der ersten Temperatur auf die zweite Temperatur entlang der operativen Länge erhöht.

2. Verfahren nach Anspruch 1, wobei das Gewichtsverhältnis zwischen Dampf und dem Kohlenwasserstoffmaterial in einem Bereich von 0,1 bis 2 liegt.

3. Verfahren nach Anspruch 1 oder Anspruch 2, wobei der Bewegtbettreaktoreintrag ferner Sauerstoff umfasst.

4. Verfahren nach einem der Ansprüche 1 bis 3, wobei der Bewegtbettreaktoreintrag ferner Wasserstoff umfasst.

5. Verfahren nach einem der Ansprüche 1 bis 4, wobei der Bewegtbettreaktoreintrag ferner recyclingfähiges Kohlenwasserstoffmaterial umfasst, wobei das Gewichtsverhältnis zwischen recyclingfähigem Kohlenwasserstoffmaterial und Kohlenwasserstoffmaterial in einem Bereich von 0,1 bis 0,75 liegt; wobei optionsweise das recyclingfähige Kohlenwasserstoffmaterial ein Produkt-Kohlenwasserstoff umfasst, und wobei das Gewichtsverhältnis zwischen Produkt-Kohlenwasserstoff und Kohlenwasserstoffmaterial in einem Bereich von 0,2 bis 0,75 liegt.

6. Direktes katalytisches Cracking-System, welches zum Umwandeln von undestilliertem und unfraktioniertem Kohlenwasserstoffmaterial in ein Kohlenwasserstoffprodukt bedienbar ist, welches eine Mischung aus leichten Olefinen und C6-8 Aromaten umfasst, unter Verwendung eines Katalysators für direktes Cracking, wobei das direkte katalytische Cracking-System Folgendes umfasst: einen Katalysator-Bewegtbettreaktor, welcher bedienbar ist, um einen Bewegtbettreaktoreintrag aufzunehmen, welcher das undestillierte und unfraktionierte Kohlenwasserstoffmaterial umfasst, und zum Transportieren eines Kohlenwasserstoffprodukts, welches eine Mischung aus leichten Olefinen und C6-8-Aromaten umfasst, welcher mehr als einen katalytischen Reaktionsbereich aufweist, wobei jeder katalytische Reaktionsbereich bedienbar ist, um den Katalysator für direktes Cracking aufzunehmen, um eine Menge an Katalysator für direktes Cracking in Form eines Katalysator-Bewegtbettes zu fassen, und um einen verbrauchten Katalysator für direktes Cracken dergestalt zu transportieren, dass er aus dem Bewegtbettreaktor austritt, welcher bedienbar ist, um das Mischen von Kohlenwasserstoffen mit dem Katalysator für direktes Cracking in dem Katalysator-Bewegtbett entlang mindestens eines Abschnitts der operativen Länge des Katalysator-Bewegtbettreaktors zu ermöglichen, und welcher Mittel zum Halten eines internen Temperaturprofils für den Katalysator-Bewegtbettreaktor entlang dessen operativer Länge aufweist; und ein Katalysator-Regenerationssystem, welches mit dem Katalysator-Bewegtbettreaktor dergestalt gekoppelt ist, dass das direkte katalytische Cracking-System bedienbar ist, um den Katalysator für direktes Cracking aus dem Katalysator-Regenerationssystem in jeden katalytischen Reaktionsbereich einzuleiten und bedienbar ist, um den verbrauchten Katalysator für direktes Cracking aus jedem katalytischen Reaktionsbereich in das Katalysator-Regenerationssystem einzuleiten, und welches bedienbar ist, um den eingeleiteten verbrauchten Katalysator für direktes Cracking in den Katalysator für direktes Cracking unter Verwendung einer Sauerstoffquelle einzuspeisen,
wobei das interne Temperaturprofil als mehr als ein fester Temperaturbereich zwischen einer Einleitungsposition des Bewegtbettreaktoreintrags und einer Position für den Transport des Kohlenwasserstoffprodukts gehalten wird, wobei ein erster Festtemperaturbereich, welcher eine erste Temperatur aufweist, unmittelbar an der Einleitungsposition positioniert ist, und ein zweiter Festtemperaturbereich, welcher eine zweite Temperatur aufweist, unmittelbar an der Position für den Transport positioniert ist,
wobei das Gerät dergestalt konfiguriert ist, dass die zweite Temperatur größer als die erste Temperatur ist und sich die Temperatur von der ersten Temperatur auf die zweite Temperatur entlang der operativen Länge erhöht; und
wobei jeder katalytische Reaktionsbereich einem der Festtemperaturbereiche zugeordnet ist, und benachbarte katalytische Reaktionsbereiche sich fluidisch miteinander durch mindestens eine zwischengeschaltete Feststoff-Abscheidungsvorrichtung koppeln.

7. Direktes katalytisches Cracking-System nach Anspruch 6, wobei der Katalysator-Bewegtbettreaktor ferner bedienbar ist, um recyclingfähiges Kohlenwasserstoffmaterial aus einem ersten Abschnitt des Katalysator-Bewegtbettreaktors in einen zweiten Abschnitt des Katalysator-Bewegtbettreaktors zu transportieren, wobei der zweite Abschnitt näher an derjenigen Position liegt, an welcher der Bewegtbettreaktoreintrag eingeleitet wird, als an der ersten Position; wobei optionsweise der erste Abschnitt eine erste Temperatur aufweist und der zweite Abschnitt eine zweite Temperatur aufweist, und wobei die erste Temperatur bei einer größeren Temperatur als der zweiten Temperatur gehalten wird.

8. Direktes katalytisches Cracking-System nach einem der Ansprüche 6 bis 7, wobei das System bedienbar ist, um einen Teil des Kohlenwasserstoffprodukts in den Bewegtbettreaktor als einen Bestandteil des Bewegtbettreaktoreintrags einzuleiten.

## Revendications

1. Procédé de conversion d'un matériau hydrocarbure non-distillé et non-fractionné en un produit hydrocarbure en utilisant un système de craquage catalytique direct, le procédé comprenant les étapes consistant à : introduire une alimentation de réacteur à lit mobile dans un réacteur à lit catalyseur mobile du système de craquage catalytique direct de sorte que l'alimentation du réacteur à lit mobile s'entremêle avec un catalyseur de craquage direct contenu dans une zone de réaction catalytique dans le réacteur à lit catalyseur mobile, dans lequel l'alimentation de réacteur à lit mobile comprend de la vapeur et un matériau hydrocarbure non distillé et non-fractionné ; introduire le catalyseur de craquage direct dans la zone de réaction catalytique du réacteur à lit catalyseur mobile ; et actionner le système de craquage catalytique direct de sorte qu'un profil de température interne soit maintenu le long d'une longueur opérationnelle du réacteur à lit catalyseur mobile, de sorte qu'un lit catalyseur mobile comprenant le catalyseur de craquage direct soit maintenu dans la zone de réaction catalytique, de sorte que le matériau hydrocarbure se convertisse en présence du catalyseur de craquage direct en un hydrocarbure de produit et le catalyseur de craquage direct se convertisse en un catalyseur de craquage direct éteint dans la zone de réaction catalytique, de sorte que le catalyseur de craquage direct éteint passe de la zone de réaction catalytique à un système de régénération de catalyseur, et de sorte que le produit hydrocarbure soit produit à partir du réacteur à lit catalyseur mobile, le produit hydrocarbure comprenant des oléfines légères et des composés aromatiques en C6-8 ; dans lequel le système de craquage catalytique direct inclut le réactif à lit catalyseur mobile et le système de régénération de catalyseur, et dans lequel le réacteur à lit catalyseur mobile présente la zone de réaction catalytique et est opérationnel pour maintenir le profil de température interne pour le réacteur à lit catalyseur mobile le long de sa longueur opérationnelle,
dans lequel le profil de température interne est maintenu comme plus d'une zone de température fixe entre une position d'introduction pour l'alimentation de réacteur à lit mobile et une position de passage pour le produit hydrocarbure, dans lequel une première zone de température fixe présentant une première température est positionnée près de la position d'introduction et une seconde zone de température fixe présentant une seconde température est positionnée près de la position de passage, et dans lequel la seconde température est supérieure à la première température, dans lequel la température augmente de la première température à la seconde température le long de la longueur opérationnelle.

2. Procédé selon la revendication 1, dans lequel le rapport en poids de la vapeur sur le matériau hydrocarbure est dans une plage allant de 0,1 à 2.

3. Procédé selon la revendication 1 ou la revendication 2, dans lequel l'alimentation de réacteur à lit mobile comprend en outre de l'oxygène.

4. Procédé selon l'une quelconque des revendications 1 à 3, dans lequel l'alimentation de réacteur à lit mobile comprend en outre de l'hydrogène.

5. Procédé selon l'une quelconque des revendications 1 à 4, dans lequel l'alimentation de réacteur à lit mobile comprend en outre un matériau hydrocarbure recyclable, dans lequel le rapport en poids du matériau hydrocarbure recyclable sur le matériau hydrocarbure est dans une plage allant de 0,1 à 0,75 ; facultativement dans lequel le matériau hydrocarbure recyclable comprend un hydrocarbure de produit, et dans lequel le rapport en poids de l'hydrocarbure de produit sur le matériau hydrocarbure est dans une plage allant de 0,2 à 0,75.

6. Système de craquage catalytique direct qui peut être actionné pour convertir un matériau hydrocarbure non-distillé et non-fractionné en un produit hydrocarbure comprenant un mélange d'oléfines légères et d'agents aromatiques en C6-8 en utilisant un catalyseur de craquage direct, le système de craquage catalytique direct comprenant : un réacteur à lit catalyseur mobile qui peut être actionné pour recevoir une alimentation de réacteur à lit mobile comprenant le matériau hydrocarbure non-distillé et non-fractionné et pour faire passer un produit hydrocarbure comprenant un mélange d'oléfines légères et d'agents aromatiques en C6-8, qui présente plus d'une zone de réaction catalytique, où chaque zone de réaction catalytique peut être actionnée pour recevoir le catalyseur de craquage direct, pour contenir une quantité du catalyseur de craquage direct sous la forme d'un lit catalyseur mobile, pour contenir une quantité du catalyseur de craquage direct sous la forme d'un lit catalyseur mobile et pour faire passer un catalyseur de craquage direct éteint de sorte qu'il ressorte du réacteur à lit catalyseur mobile, qui peut être actionné pour permettre le brassage d'hydrocarbures avec le catalyseur de craquage direct dans le lit catalyseur mobile le long d'au moins une partie de la longueur opérationnelle du réacteur à lit catalyseur mobile, et présentant des moyens permettant de maintenir un profil de température interne pour le réacteur à lit catalyseur mobile le long de sa longueur opérationnelle ; et un système de régénération de catalyseur qui se raccorde au réacteur à lit catalyseur mobile de sorte que le système de craquage catalytique direct puisse être actionné pour introduire le catalyseur de craquage direct depuis le système de régénération de catalyseur vers chaque zone de réaction catalytique et puisse être actionné pour introduire le catalyseur de craquage direct éteint de chaque zone de réaction catalytique vers le système de régénération de catalyseur, et qu'il puisse être actionné pour convertir le catalyseur de craquage direct éteint introduit dans le catalyseur de craquage direct en utilisant une source d'oxygène,
dans lequel le profil de température interne est maintenu comme plus d'une zone de température fixe entre une position d'introduction pour l'alimentation du réacteur à lit mobile et une position de passage pour le produit hydrocarbure, dans lequel une première zone de température fixe présentant une première température est positionnée près de la position d'introduction et une seconde zone de température fixe présentant une seconde température est positionnée près de la position de passage,
dans lequel l'appareil est configuré de sorte que la seconde température soit supérieure à la première température et la température augmente de la première température à la seconde température le long de la longueur opérationnelle ; et
dans lequel chaque zone de réaction catalytique est associée à une des zones de température fixes et des zones de réaction catalytiques adjacentes se relient fluidiquement les unes avec les autres à travers au moins un dispositif de séparation de solides intermédiaire.

7. Système de craquage catalytique direct selon la revendication 6, dans lequel le réacteur à lit catalyseur mobile peut en outre être actionné pour faire passer un matériau hydrocarbure recyclable d'une première partie du réacteur à lit catalyseur mobile vers une seconde partie du réacteur à lit catalyseur mobile, dans lequel la seconde partie est plus proche de la position dans laquelle l'alimentation de réacteur à lit mobile est introduite que la première position, facultativement dans lequel la première partie présente une première température et la seconde partie présente une seconde température et dans lequel la première température est maintenue à une température supérieure à la seconde température.

8. Système de craquage catalytique direct selon l'une quelconque des revendications 6 à 7, dans lequel le système peut être actionné pour introduire une partie du produit hydrocarbure dans le réacteur à lit mobile dans le cadre de l'alimentation du réacteur à lit mobile.
